Europäisches Patentamt

European Patent Office

Office européen des brevets

(11) Publication number: **0 606 847 A2**

## EUROPEAN PATENT APPLICATION

(21) Application number: **94100104.2**

(22) Date of filing: **05.01.94**

(51) Int. Cl.5: **C12N 5/06**

(30) Priority: **07.01.93 JP 1257/93**

(43) Date of publication of application:
**20.07.94 Bulletin 94/29**

(84) Designated Contracting States:
**DE DK FR GB**

(71) Applicant: **NATIONAL INSTITUTE OF ANIMAL INDUSTRY, MINISTRY OF AGRICULTURE, FORESTRY AND FISHERIES**
**2, Ikenodai,**
**Kukizaki-machi**
**Inashiki-gun, Ibaraki(JP)**
Applicant: **MEIJI SEIKA KAISHA LTD.**
**4-16 Kyobashi 2-chome**
**Chuo-ku Tokyo(JP)**

(72) Inventor: **Nagai, Takashi**
**125-203, Namiki 2-chome**
**Tsukuba-shi, Ibaraki(JP)**
Inventor: **Takenaka, Akio**
**2-5-1-504, Inodai**
**Toride-shi, Ibaraki(JP)**
Inventor: **Hirayama, Masao, c/o Meiji Seika Kaisha, Ltd.**
**5-3-1, Chiyoda**
**Sakado-shi, Saitama(JP)**
Inventor: **Kamo, Yoshihiro, c/o Meiji Seika Kaisha, Ltd.**
**5-3-1, Chiyoda**
**Sakado-shi, Saitama(JP)**
Inventor: **Nishizawa, Koji, c/o Meiji Seika Kaisha, Ltd.**
**5-3-1, Chiyoda**
**Sakado-shi, Saitama(JP)**

(74) Representative: **Kraus, Walter, Dr. et al**
**Patentanwälte Kraus, Weisert & Partner**
**Thomas-Wimmer-Ring 15**
**D-80539 München (DE)**

(54) **Medium for in vitro fertilization and method of in vitro fertilization.**

(57) A medium for in vitro fertilization of a mammal which comprises casein phosphopeptide, and a method of in vitro fertilization of a mammal comprising carrying out the in vitro fertilization in a culture solution comprising a medium for in vitro fertilization of the mammal containing casein phosphopeptide. According to the invention, a fertilization rate in vitro can be improved and stabilized irrespective of source of spermatozoa.

## BACKGROUND OF THE INVENTION

This invention relates to a medium for in vitro fertilization of a mammal which improves in vitro fertilization rate and a method of in vitro fertilization of using the same.

Recently, in mammals, fertilization of oocytes have widely carried out in vitro for breeding and multiplication of elite species and the like. In the in vitro fertilization, immature oocytes are recovered from the ovary of living body or slaughtered body of a mammal by suction, the oocytes are cultured in a medium for maturing in vitro, and then, inseminated with fresh or frozen-thawed spermatozoa when the oocytes have matured. After the fertilization, the oocytes are cultured by tranferring to a medium for the development, and meanwhile, embryos are transferred to an animal who receives the embryos so as to conform with her estrus. This technique is very excellent in principle. However, there are extremely low fertilization rate cases according to spermatozoa, and an in vitro fertilization wherein every spermatozoon can penetrate an oocyte has not established yet.

Thereupon, various improvements in culture media and treatment of spermatozoa have been made in order to establish a fertilization system effective for every spermatozoon in the in vitro fertilization, but such a fertilization system has not been established yet, and further improvement is desired.

## SUMMARY OF THE INVENTION

An object of the invention is to provide a medium for in vitro firtilization of a mammal effective for every spermatozoon and a method of in vitro fertilization using the same.

The present inventors have investigated in order to establish a fertilization system capable of carrying out in vitro fertilization stably irrespective of the source of spermatozoa, and conventional unstable fertilization is caused by no occurrence of the acrosome reaction which is necessary for the penetration of a spermatozoon into an oocyte due to insufficient intake of calcium into the spermatozoon in the conventional fertilization medium by taking the fertilization mechanism of a mammal into consideration. However, calcium is an essential component for the acrosome reaction of a spermatozoon, and in general, calcium is sufficiently incorporated into the conventional medium for fertilization in an amount more than the necessary amount. Under the conditions used as a culture solution for fertilization, there is a limit to increase the blending amount of calcium, and moreover, the effect on the improvement in the fertilization rate is not so great by increasing the calcium component. Then, the inventors have further investigated, and found that the fertilization rate can be improved by adding casein phosphopeptide to an in vitro fertilization medium.

Thus, the present invention provides a medium for in vitro fertilization of a mammal which contains casein phosphopeptide and a method of in vitro fertilization of a mammal comprising carring out the in vitro fertilization in a culture solution containing the medium.

## DETAILED DESCRIPTION OF THE INVENTION

Casein phosphopeptide is a hydrolyzed product of casein, and is a phosphopeptide having an activity to solubilize calcium. It has been reported that casein taken as a food is digested in the intestinal tract to produce casein phosphopeptide, and the casein phosphopeptide increases the absorption of calcium at the intestine by solubilizing calcium (Nippon Eiyo, Shokuryo Gakkai-shi, vol. 36, pp 433-439, 1986).

As casein, there are $\alpha$-casein, $\beta$-casein, $\gamma$-casein, and the like, and every casein can be used. As to casein phosphopeptide, there are several reports, and in the case of digestion products by trypsin, it is known to produce the peptide chain of 42 to 79 residues of $\alpha_{s1}$-casein, the peptide chains of 1 to 25 and 1 to 28 residues of $\beta$-casein and the peptide chain of 1 to 32 residues of $\alpha_{s2}$-casein. The casein phosphopeptide usable for the medium for in vitro fertilization of the invention may be any casein phosphopeptide having an activity to solubilize calcium.

It is disclosed that the active site to solubilize casein is the phosphoserine portion of casein phosphopeptide, and the molecular weight may be not more than 10,000. The means for decomposing casein to produce casein phosphopeptide is not limited, but digestion products by a protease not decomposing the active site, such as pepsin and trypsin, are preferred.

A suitable casein phosphopeptide content of the medium for in nitro fertilization is about 0.1 to 30 % (weight), preferably about 0.8 to 12 %, and as the culture solution prepared using the medium, a suitable casein phosphopeptide content is about 0.005 to 1 % (weight/volume), preferably about 0.02 to 0.3 %.

As the other medium components, it is necessary to contain water-soluble calcium, such as calcium chloride or calcium lactate. A suitable water-soluble calcium content of the medium is about 0.03 to 3 % (as the weight of calcium), preferably about 0.1 to 1 %. Media for in vitro fertilization, in general, contain water-

2

soluble calcium. When the content of the water-soluble calcium is in the above range, it is not necessary further to add water-soluble calcium to the medium.

It is preferable to contain caffeine in the medium. By adding caffeine to the medium, the fertilization rate can be further improved. A suitable content of caffeine in the medium is about 0.1 to 20 % (weight), preferably about 0.2 to 12 %, and a suitable content of caffeine in a culture solution is 0.005 to 1 % (weight/volume), preferably 0.05 to 0.3 %.

The medium components other than above-mentioned may be similar to a conventional medium for in vitro fertilization which contains fundamental inorganic components, such as Na, K, Mg, P and Cl, serum or serum components such as BSA or the like according to metablic ability, carbonate, glucose, pyruvic acid, and the like. As examples of the conventional medium, there are BO solution for in vitro fertilization, TALP solution and the like.

The medium can be prepared similar to the conventional medium, and casein phosphopeptide may be added together with other medium components or separately added.

The in vitro fertilization using the above-metioned medium can be carried out, for example, as follows:

Porcine ova matured in a commercial Waymouth solution or the like are transferred to a culture solution containing 0.005 to 1 % (weight/volume) of casein phosphopeptide prepared from a known medium for in vitro fertilization, such as BO solution for in vitro fertilization, to which casein phosphopeptide has been added in an amount of 0.1 to 30 % (weight). Frozen-thawed epididymal spermatozoa are added to the culture solution, and cultured under known conditions. Although a fertilization rate to a certain degree can be obtained by using the conventional medium, the fertilization rate can be improved by using the medium for in vitro fertilization of the invention containing casein phosphopeptide by the calcium solubilization mechanism.

As application embodiment of the medium of the invention to swine is mentioned above, and the medium can be applied to other mammals, such as cattle, horse, sheep and human, utilizing similar principle. That is, casein phosphopeptide is added to a known medium for in vitro fertilization suitable for each mammal, and a culture solution containing casein phosphopeptide is prepared therefrom. Then, the in vitro fertilization is carried out in the culture solution.

According to the invention, a fertilization rate in vitro can be improved and stabilized irrespective of spermatozoa.

EXAMPLES

Example 1

The BO solution for in vitro fertilization (IVF) used has the composition of Table 1.

Table 1

Composition of BO Medium for IVF

| Component | | Blending Amount |
|---|---|---|
| A-Solution | | 76 ml |
| NaCl | 4.309 g / 500 ml | |
| KCl | 0.197 g / 500 ml | |
| $CaCl_2 \cdot 2H_2O$ | 0.217 g / 500 ml | |
| $NaH_2PO_4 \cdot 2H_2O$ | 0.084 g / 500 ml | |
| $MgCl_2 \cdot 6H_2O$ | 0.070 g / 500 ml | |
| Phenol Red | 1.0 mg / 500 ml | |
| B-Solution | | 24 ml |
| $NaHCO_3$ | 2.587 g / 200 ml | |
| Phenol Red | 0.4 mg / 200 ml | |
| Glucose | | 0.25 g |
| Sodium Pyruvate | | 3.8 mg |
| Bovine Serum Albumin (BSA) | | 1.0 g |

To the BO solution, casein phosphopeptide ("CPP", Meiji Seika Kaisha, Ltd.) was added in an amount of 1 mg/ml, and to the BO solution to which casein phosphopeptide had been added or not added, caffeine was added in an amount of 0.5 or 15 mM to prepare 6 kinds of culture solutions for fertilization. Porcine ova matured in a commercial Waymouth solution (containing 10 % overian follicle solution, 10 % fetal bovine serum, luteinizing hormone and follicle-stimulating hormone) were transferred to each of the above 6 kinds of culture solutions for fertilization, and fused matter of lyophilized spermatozoa in the epididymis (swine A) was added so that the final concentration became $1 \times 10^5$ cells/ml, and cultured at 39 °C for 6 hours. The results of fertilization rate are shown in Table 2.

Table 2

| Effect of CPP on Fertilization of Porcine Oocytes | | | | | |
|---|---|---|---|---|---|
| Group | Caffeine (mM) | CPP (mg/ml) | Number of Ova | | Fertilization Rate (%) |
| | | | Tested | Fertilized | |
| 1 | 0 | 0 | 24 | 0 | 0 |
| 2 | 0 | 1 | 26 | 1 | 4 |
| 3 | 5 | 0 | 56 | 25 | 45 |
| 4 | 5 | 1 | 48 | 40 | 83 |
| 5 | 15 | 0 | 58 | 34 | 59 |
| 6 | 15 | 1 | 53 | 52 | 98 |

As shown is Table 2, the fertilization rate in the medium added with CPP was significantly higher compared with the corresponding no CPP added group.

Example 2

Three kinds of culture solutions were prepared, which were the same culture solution as Group 4 of Example 1, i.e. 1 mg/ml of CPP and 5 mM of caffeine were added, (Group 7), the same culture solution as the above Group 7 except adding 1 mM of dephosphorized CPP which was prepared by removing phosphate group from the CPP by treating with phosphatase instead of the CPP as a comparative example (Group 8), and the same culture solution as the above Group 7 except not adding the CPP (Group 9). Similar to Example 1, porcine ova were cultured in each culture solution at 39 °C for 46 to 48 hours, and then, frozen-thawed epididymal spermatozoa of three swines (A, B, C) was separately added. The results of the rate of ova into which at least one spermatozoon penetrated and the rate of ova into which a plurality of spermatozoa penetrated are shown in Table 3.

Table 3

| Effect of CPP on Spermatozoon Penetration into Porcine Ovum | | | | | | |
|---|---|---|---|---|---|---|
| Swine | Group | CPP (mg/ml) | De-P CPP (mg/ml) | Number of Oocytes | | |
| | | | | Tested | Spermatozoon Penetration (%) | Spermatozoa(pl.) Penetration (%) |
| A | 7 | 1 | 0 | 59 | 51(86) | 35(68) |
| | 8 | 0 | 1 | 56 | 29(52) | 9(31) |
| | 9 | 0 | 0 | 59 | 23(39) | 7(30) |
| B | 7 | 1 | 0 | 63 | 41(65) | 17(41) |
| | 8 | 0 | 1 | 59 | 14(24) | 3(21) |
| | 9 | 0 | 0 | 64 | 10(16) | 3(30) |
| C | 7 | 1 | 0 | 55 | 50(91) | 36(72) |
| | 8 | 0 | 1 | 58 | 35(60) | 15(42) |
| | 9 | 0 | 0 | 59 | 17(29) | 7(41) |

As shown in Table 3, both of the rate of ova into which at least one spermtozoon penetrated and the rate of ova into which a plurality of spermatozoa penetrated of Group 1 (CPP added group) were significantly higher compared with Group 2 (dephosphorized CPP added group) and Group 3 (no CPP added group).

**Claims**

1.  A medium for in vitro fertilization of a mammal which comprises casein phosphopeptide.

2.  The medium of claim 1 which further comprises fundamental inorganic components and a serum component.

3.  The medium of claim 1 wherein the content of casein phosphopeptide is 0.1 to 30 % by weight.

4.  The medium of claim 2 which further comprises 0.1 to 20 % by weight of caffeine.

5.  The medium of claim 1 or 2 which is BO solution or TALP solution for in vitro fertilization to which 0.1 to 30 % by weight of casein phosphopeptide is added.

6.  The medium of claim 1 or 2 wherein the mammal is a member selected from the group consisting of swine, cattle, horse and sheep.

7.  The medium of claim 1 or 2 wherein the mammal is swine.

8. A method of in vitro fertilization of a mammal comprising carrying out the in vitro fertilization in a culture solution comprising a medium for in vitro fertilization of the mammal containing casein phosphopeptide.

9. The method of calim 8 wherein the casein phosphopeptide content of the culture solution is 0.005 to 1 % by weight/volume ratio.

10. The method of claim 8 wherein the calture solution further comprises 0.005 to 1 % by weight/volume ratio of caffeine.

11. The method of claim 8 wherein a frozen-thawed spermatozoa of the mammal is used.

12. The method of claim 8 wherein the mammal is swine.